(19) Europäisches Patentamt
European Patent Office
Office européen des brevets

(11) Veröffentlichungsnummer: **0 334 133 A1**

(12) # EUROPÄISCHE PATENTANMELDUNG

(21) Anmeldenummer: **89104336.6**

(51) Int. Cl.4: **C07D 403/04 , A01N 43/56**

(22) Anmeldetag: **11.03.89**

(30) Priorität: **25.03.88 DE 3810101**

(43) Veröffentlichungstag der Anmeldung: **27.09.89 Patentblatt 89/39**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Santel, Hans-Joachim, Dr.**
**Grünstrasse 9 a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6 a**
**D-4000 Düsseldorf 31(DE)**

(54) **Pyrazolylpyrrolinone.**

(57) Die Erfindung betrifft neue Pyrazolylpyrrolinone der Formel (I),

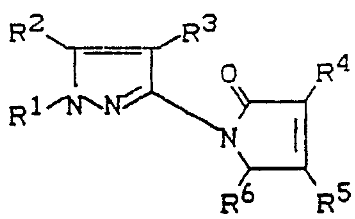

(I)

in welcher
$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,
$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,
$R^3$ für Wasserstoff oder Alkyl steht,
$R^4$ und $R^5$ entweder unabhängig voneinander jeweils für Wasserstoff oder Alkyl stehen oder gemeinsam für einen jeweils zweifach verknüpften Alkandiyl-, Alkendiyl- oder Alkdiendiylrest stehen und
$R^6$ für Halogen, Hydroxy, Mercapto, Amino, Alkylamino, Dialkylamino, für einen Rest $R^7-\underset{\underset{O}{\|}}{C}-O-$
oder für einen Rest $R^8-SO_2-O-$ steht,
mehrere Verfahren sowie neue Zwischenprodukte zu deren Herstellung und ihre Verwendung als Herbizide.

EP 0 334 133 A1

## Pyrazolylpyrrolinone

Die Erfindung betrifft neue Pyrazolylpyrrolinone, mehrere Verfahren zu ihrer Herstellung und ihre Verwendung als Herbizide.

Es ist bekannt, daß bestimmte heterocyclisch substituierte Pyrrolinone, wie beispielsweise die Verbindung 3,4-Dimethyl-5-hydroxy-1-(5-trifluormethyl-1,3,4-thiadiazol-2-yl)-$\Delta^3$-pyrrolin-2-on herbizide Eigenschaften besitzen (vergl. z.B. DE-OS 27 35 841).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Pyrazolylpyrrolinone der allgemeinen Formel (I),

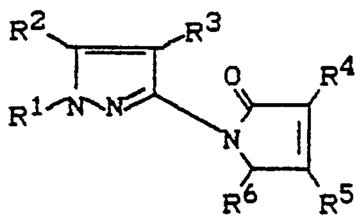

(I)

in welcher

$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,

$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,

$R^3$ für Wasserstoff oder Alkyl steht,

$R^4$ und $R^5$ entweder unabhängig voneinander jeweils für Wasserstoff oder Alkyl stehen oder gemeinsam für einen jeweils zweifach verknüpften Alkandiyl-, Alkendiyl- oder Alkdiendiylrest stehen und

$R^6$ für Halogen, Hydroxy, Mercapto, Amino, Alkylamino, Dialkylamino, für einen Rest $R^7-\underset{\underset{O}{\|}}{C}-O-$ oder für einen Rest $R^8-SO_2-O-$ steht,

wobei

$R^7$ für Wasserstoff, Alkyl, Alkenyl, Alkinyl, Halogenalkyl, Alkoxyalkyl, Alkylthioalkyl, Alkylcarbonylalkyl, Alkoxycarbonylalkyl, für Alkylamino, für Dialkylamino oder für jeweils gegebenenfalls substituiertes Cycloalkyl oder Aryl steht und

$R^8$ für Alkyl, Halogenalkyl, Alkylamino, Dialkylamino oder für gegebenenfalls substituiertes Aryl steht,

gefunden.

Weiterhin wurde gefunden, daß man die neuen Pyrazolylpyrrolinone der allgemeinen Formel (I),

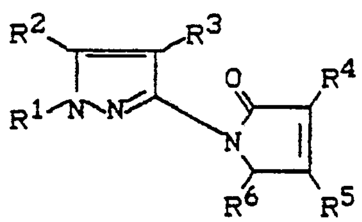

(I)

in welcher

$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,

$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,

$R^3$ für Wasserstoff oder Alkyl steht,

$R^4$ und $R^5$ entweder unabhängig voneinander jeweils für Wasserstoff oder Alkyl stehen oder gemeinsam für einen jeweils zweifach verknüpften Alkandiyl-, Alkendiyl- oder Alkdiendiylrest stehen und

$R^6$ für Halogen, Hydroxy, Mercapto, Amino, Alkylamino, Dialkylamino, für einen Rest $R^7-\underset{\underset{O}{\|}}{C}-O-$

oder für einen Rest $R^8$-$SO_2$-O- steht,
wobei
$R^7$ für Wasserstoff, Alkyl, Alkenyl, Alkinyl, Halogenalkyl, Alkoxyalkyl, Alkylthioalkyl, Alkylcarbonylalkyl, Alkoxycarbonylalkyl, für Alkylamino, für Dialkylamino oder für jeweils gegebenenfalls substituiertes Cycloalkyl oder Aryl steht und
$R^8$ für Alkyl, Halogenalkyl, Alkylamino, Dialkylamino oder für gegebenenfalls substituiertes Aryl steht,
nach einem der im Folgenden beschriebenen Verfahren erhält:

(a) Man erhält Pyrazolylpyrrolinone der Formel (Ia),

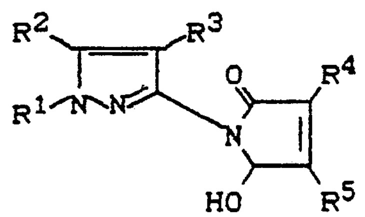

(Ia)

in welcher
$R^1$, $R^2$, $R^3$, $R^4$ und $R^5$ die oben angegebene Bedeutung haben,
wenn man Pyrazolylpyrrolindione der Formel (II),

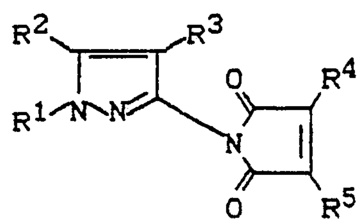

(II)

in welcher
$R^1$, $R^2$, $R^3$, $R^4$ und $R^5$ die oben angegebene Bedeutung haben,
mit einem Reduktionsmittel gegebenenfalls in Gegenwart eines Verdünnungsmittels umsetzt;

(b) man erhält Pyrazolylpyrrolinone der Formel (Ib),

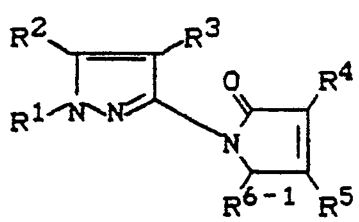

(Ib)

in welcher
$R^{6-1}$ für Halogen, Mercapto, Amino, Alkylamino oder Dialkylamino steht und
$R^1$, $R^2$, $R^3$, $R^4$ und $R^5$ die oben angegebene Bedeutung haben,
wenn man die mit Hilfe des erfindungsgemäßen Verfahrens (a) erhältlichen Pyrazolylpyrrolinone der Formel (Ia),

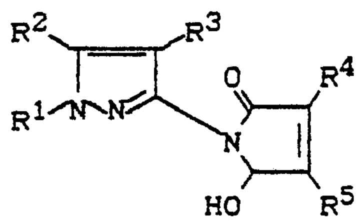

(Ia)

in welcher
$R^1$, $R^2$, $R^3$, $R^4$ und $R^5$ die oben angegebene Bedeutung haben,
mit nukleophilen Reagenzien der Formel (III),

$R^{6-1}$-A     (III)

in welcher
A für Wasserstoff, für ein Alkalimetallkation oder für einen anderen geeigneten Abgangsrest steht und
$R^{6-1}$ die oben angegebene Bedeutung hat,
gegebenenfalls in Gegenwart eines Verdünnungsmittels und gegebenenfalls in Gegenwart eines Reaktionshilfsmittels umsetzt;

(c) man erhält Pyrazolylpyrrolinone der Formel (Ic),

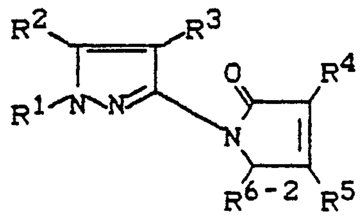

(Ic)

in welcher
$R^{6-2}$ für einen Rest $R^7-\underset{\underset{O}{\|}}{C}-O-$
oder für einen Rest $R^8$-$SO_2$-O- steht und
$R^1$, $R^2$, $R^3$, $R^4$, $R^5$, $R^7$ und $R^8$ die oben angegebene Bedeutung haben,
wenn man die mit Hilfe des erfindungsgemäßen Verfahrens (a) erhältlichen Pyrazolylpyrrolinone der Formel (Ia),

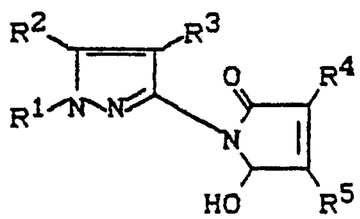

(Ia)

in welcher
$R^1$, $R^2$, $R^3$, $R^4$ und $R^5$ die oben angegebene Bedeutung haben,
entweder

(α) mit Acylierungsmitteln der Formel (IV),

$R^7-\underset{\underset{O}{\|}}{C}-E^1$     (IV)

in welcher
$E^1$ für eine elektronenanziehende Abgangsgruppe steht und
$R^7$ die oben angegebene Bedeutung hat oder

(β) mit Isocyanaten der Formel (V),

$R^{7-1}$-N=C=O     (V)

in welcher
$R^{7-1}$ für Alkyl steht oder

(γ) mit Sulfonylierungsmitteln der Formel (VI),

$R^8$-$SO_2$-$E^2$     (VI)

in welcher
$E^2$ für eine elektronenanziehende Abgangsgruppe steht und
$R^8$ die oben angegebene Bedeutung hat,
jeweils gegebenenfalls in Gegenwart eines Verdünnungsmittels und gegebenenfalls in Gegenwart eines Reaktionshilfsmittels umsetzt.

Schließlich wurde gefunden, daß die neuen Pyrazolylpyrrolinone der allgemeinen Formel (I) herbizide

Wirksamkeit besitzen.

Überraschenderweise zeigen die erfindungsgemäßen Pyrazolylpyrrolinone der allgemeinen Formel (I) eine erheblich bessere herbizide Wirksamkeit gegenüber Problemunkräutern und gleichzeitig eine deutlich verbesserte Verträglichkeit gegenüber wichtigen Kulturpflanzen im Vergleich zu den aus dem Stand der Technik bekannten heterocyclisch substituierten Pyrrolinonen, wie beispielsweise die Verbindung 3,4-Dimethyl-5-hydroxy-1-(5-trifluormethyl-1,3,4-thiadiazol-2-yl)-$\Delta^3$-pyrrolin-2-on, welche chemisch und wirkungsmäßig naheliegende Verbindungen sind.

Die erfindungsgemäßen Pyrazolylpyrrolinone sind durch die Formel (I) allgemein definiert. Bevorzugt sind Verbindungen der Formel (I), bei welchen

$R^1$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Alkoxyalkyl mit jeweils 1 bis 4 Kohlenstoffatomen in den einzelnen Alkylteilen oder für jeweils gegebenenfalls einfach oder mehrfach, gleich oder verschieden substituiertes Benzyl, Phenylethyl oder Phenyl steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen sowie jeweils geradkettiges oder verzweigtes Alkoxycarbonyl oder Alkoximinoalkyl mit jeweils 1 bis 4 Kohlenstoffatomen in den einzelnen Alkylteilen;

$R^2$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Alkoxyalkyl oder Alkylthioalkyl mit jeweils 1 bis 4 Kohlenstoffatomen in den einzelnen Alkylteilen und 1 bis 3 Alkoxy- bzw. Alkylthiogruppen oder für gegebenenfalls einfach oder mehrfach, gleich oder verschieden substituiertes Cycloalkyl mit 3 bis 7 Kohlenstoffatomen steht, wobei als Substituenten jeweils infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen; oder $R^2$ für gegebenenfalls einfach oder mehrfach, gleich oder verschieden substituiertes Phenyl steht, wobei als Substituenten die bei $R^1$ genannten infrage kommen;

$R^3$ für Wasserstoff oder für geradkettiges oder verzweigtes Alkyl mit 1 bis 4 Kohlenstoffatomen steht,

$R^4$ und $R^5$ entweder unabhängig voneinander jeweils für Wasserstoff oder für geradkettiges oder verzweigtes Alkyl mit 1 bis 4 Kohlenstoffatomen stehen oder gemeinsam für einen jeweils zweifach verknüpften Butan-1,4-diylrest, Buten-1,4-diylrest oder Butadien-1,4-diylrest stehen und

$R^6$ für Fluor, Chlor, Brom, Iod, Hydroxy, Mercapto, Amino, für jeweils geradkettiges oder verzweigtes Alkylamino oder Dialkylamino mit jeweils 1 bis 4 Kohlenstoffatomen in den einzelnen Alkylteilen, für einen Rest $R^7-\underset{\underset{O}{\|}}{C}-O-$

oder für einen Rest $R^8-SO_2-O-$steht, wobei

$R^7$ für Wasserstoff, für jeweils geradkettiges oder verzweigtes Alkyl mit 1 bis 6 Kohlenstoffatomen, Alkenyl mit 2 bis 6 Kohlenstoffatomen, Alkinyl mit 2 bis 6 Kohlenstoffatomen, Halogenalkyl mit 1 bis 6 Kohlenstoffatomen und 1 bis 13 gleichen oder verschiedenen Halogenatomen, Alkoxyalkyl, Alkylthioalkyl, Alkylcarbonylalkyl oder Alkoxycarbonylalkyl mit jeweils 1 bis 4 Kohlenstoffatomen in den einzelnen Alkylteilen, Alkylamino oder Dialkylamino mit jeweils 1 bis 4 Kohlenstoffatomen in den einzelnen Alkylteilen oder für gegebenenfalls einfach oder mehrfach, gleich oder verschieden substituiertes Cycloalkyl mit 3 bis 7 Kohlenstoffatomen steht, wobei als Substituenten jeweils infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen; oder für gegebenenfalls einfach oder mehrfach, gleich oder verschieden substituiertes Phenyl steht, wobei als Substituenten die bei $R^1$ genannten infrage kommen und

$R^8$ für jeweils geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, Alkylamino oder Dialkylamino mit jeweils 1 bis 4 Kohlenstoffatomen in den einzelnen Alkylteilen oder für gegebenenfalls einfach oder mehrfach, gleich oder verschieden substituiertes Phenyl steht, wobei als Substituenten die bei $R^1$ genannten infrage kommen.

Besonders bevorzugt sind Verbindungen der Formel (I), bei welchen

$R^1$ für Wasserstoff, Methyl, Ethyl, n- oder i-Propyl, n-, i-, s- oder t-Butyl, für Allyl, für n- oder i-Butenyl, für Propargyl, für n- oder i-Butinyl, für Fluormethyl, Difluormethyl, Trifluormethyl, Chlormethyl, Dichlormethyl,

Trichlormethyl, Dichlorfluormethyl, Chlordifluormethyl, für Chlorallyl, Dichlorallyl, Chlorbutenyl, Brombutenyl, für Methoxymethyl, Ethoxymethyl, n- oder i-Propoxy methyl, Methoxyethyl, Ethoxyethyl, n- oder i-Propoxyethyl oder für jeweils gegebenenfalls ein-bis dreifach, gleich oder verschieden substituiertes Benzyl oder Phenyl steht, wobei als Substituenten infrage kommen: Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Trifluormethyl, Difluormethyl, Trifluormethoxy, Difluormethoxy, Trifluormethylthio, Methoxycarbonyl, Ethoxycarbonyl, Methoximinomethyl, Ethoximinomethyl, Methoximinoethyl oder Ethoximinoethyl;

$R^2$ für Wasserstoff, Methyl, Ethyl, n- oder i-Propyl, n-, i-, s- oder t-Butyl, für jeweils geradkettiges oder verzweigtes Pentyl, Hexyl oder Heptyl, für Allyl, für jeweils geradkettiges oder verzweigtes Butenyl, Pentenyl oder Hexenyl, für Propargyl, für jeweils geradkettiges oder verzweigtes Butinyl, Pentinyl oder Hexinyl, für jeweils geradkettiges oder verzweigtes Halogenalkyl mit 1 bis 6 Kohlenstoffatomen und 1 bis 3 gleichen oder verschiedenen Halogenatomen - insbesondere Fluor, Chlor oder Brom- oder Halogenalkenyl mit 2 bis 6 Kohlenstoffatomen und 1 bis 3 gleichen oder verschiedenen Halogenatomen - insbesondere Fluor, Chlor oder Brom-, für jeweils geradkettiges oder verzweigtes Alkoxyalkyl oder Alkylthioalkyl mit jeweils 1 bis 4 Kohlenstoffatomen in den einzelnen Alkylteilen und 1 bis 3 Alkoxy- bzw. Alkylthiogruppen, für jeweils gegebenenfalls ein- bis fünffach, gleich oder verschieden durch Fluor, Chlor, Brom, Methyl, Ethyl oder Trifluormethyl substituiertes Cyclopropyl, Cyclobutyl, Cyclopentyl oder Cyclohexyl oder für gegebenenfalls ein- bis dreifach, gleich oder verschieden substituiertes Phenyl steht, wobei als Substituenten die bei $R^1$ genannten infrage kommen;

$R^3$ für Wasserstoff, Methyl oder Ethyl steht,

$R^4$ und $R^5$ entweder unabhängig voneinander jeweils für Wasserstoff, Methyl oder Ethyl stehen oder gemeinsam für einen Butan-1,4-diylrest oder einen Butadien-1,4-diylrest stehen und

$R^6$ für Chlor, Brom, Iod, Hydroxy, Mercapto, für Amino, Methylamino, Ethylamino, Dimethylamino oder Diethylamino oder für einen Rest

$$R^7-\underset{\underset{O}{\|}}{C}-O-$$

oder für einen Rest $R^8-SO_2-O-$steht, wobei

$R^7$ für Wasserstoff, Methyl, Ethyl, n- oder i-Propyl, für Vinyl, Allyl, Ethinyl, Propargyl, für Chlormethyl, Dichlormethyl, Trichlormethyl, 1-Chlorethyl, 2-Chlorethyl, 2-Bromethyl, 1-Bromethyl, Trifluormethyl, Difluormethyl, für Methoxymethyl, Ethoxymethyl, Methoxyethyl, Ethoxyethyl, Methylthiomethyl, Methylcarbonylmethyl, Ethylcarbonylmethyl, Methoxycarbonylmethyl, Ethoxycarbonylmethyl, für Methylamino, Ethylamino, i-Propylamino, Dimethylamino, Diethylamino, für Cyclopropyl, Cyclobutyl, Cy clopentyl, Cyclohexyl, oder für gegebenenfalls ein- bis dreifach, gleich oder verschieden substituiertes Phenyl steht, wobei als Substituenten die bei $R^1$ genannten infrage kommen und

$R^8$ für Methyl, Ethyl, n- oder i-Propyl, Trifluormethyl, Methylamino, Ethylamino, Dimethylamino, Diethylamino oder für gegebenenfalls ein- bis dreifach, gleich oder verschieden substituiertes Phenyl steht, wobei als Substituenten die bei $R^1$ genannten infrage kommen.

Ganz besonders bevorzugt sind Verbindungen der Formel (I), bei welchen

$R^1$ für Wasserstoff, Methyl, Ethyl, n- oder i-Propyl, n-, i-, s- oder t-Butyl, Allyl, Propargyl, Chlorallyl, für Difluormethyl, Ethoxyethyl oder für jeweils gegebenenfalls einfach oder zweifach, gleich oder verschieden durch Fluor, Chlor, Methyl, Methoxy oder Trifluormethyl substituiertes Benzyl oder Phenyl steht,

$R^2$ für Wasserstoff, Methyl, Ethyl, i-Propyl, i-, s-oder t-Butyl, jeweils verzweigtes Pentyl, Hexyl oder Heptyl, verzweigtes Hexenyl, jeweils verzweigtes Alkoxyalkyl oder Alkylthioalkyl mit jeweils 1 bis 4 Kohlenstoffatomen in den einzelnen Alkylteilen und 1 bis 3 Alkoxy- bzw. Alkylthiogruppen, verzweigtes Halogenalkyl mit 1 bis 4 Kohlenstoffatomen und 1 bis 3 gleichen oder verschiedenen Halogenatomen - insbesondere Fluor, Chlor oder Brom - oder für jeweils gegebenenfalls ein- bis fünffach, gleich oder verschieden durch Fluor, Chlor, Methyl oder Trifluormethyl substituiertes Cyclopropyl, Cyclobutyl oder Cyclohexyl oder für gegebenenfalls einfach oder zweifach, gleich oder verschieden durch Fluor, Chlor, Methyl, Methoxy oder Trifluormethyl substituiertes Phenyl steht,

$R^3$ für Wasserstoff oder Methyl steht,

$R^4$ und $R^5$ für Methyl stehen oder gemeinsam für einen Butan-1,4-diylrest oder einen Butadien-1,4-diylrest stehen und

$R^6$ für Chlor, Hydroxy oder für einen Rest $R^7-\underset{\underset{O}{\|}}{C}-O-$

oder $R^8-SO_2-O-$ steht,

wobei

$R^7$ für Methyl, Ethyl, Vinyl, Chlormethyl, Dichlormethyl, Trifluormethyl, 1-Chlorethyl, Methylcarbonylmethyl, für Cyclopropyl, Cyclohexyl, für Methylamino, für Dimethylamino oder für gegebenenfalls einfach oder

zweifach, gleich oder verschieden durch Fluor, Chlor, Methyl, Methoxy oder Trifluormethyl substituiertes Phenyl steht und

$R^8$ für Methyl, Trifluormethyl, Methylamino, Dimethylamino oder für gegebenenfalls einfach oder zweifach, gleich oder verschieden durch Fluor, Chlor, Methyl, Methoxy oder Trifluormethyl substituiertes Phenyl steht.

Im einzelnen seien außer den bei den Herstellungsbeispielen genannten Verbindungen die folgenden Pyrazolylpyrrolinone der allgemeinen Formel (I) genannt:

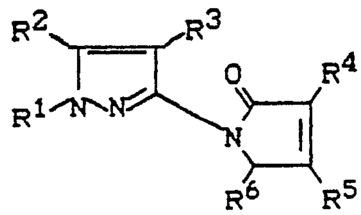

(I)

| $R^1$ | $R^2$ | $R^3$ | 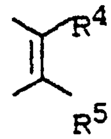 | $R^6$ |
|---|---|---|---|---|
| H | $(CH_3)_3C-$ | H | 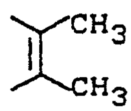 | Cl |
| H | $(CH_3)_3C-$ | H | 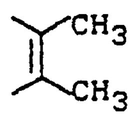 | 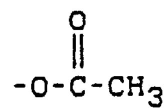 |
| H | $(CH_3)_3C-$ | H | 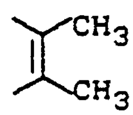 | 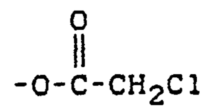 |
| H | $(CH_3)_3C-$ | H | 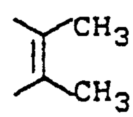 | 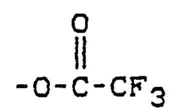 |

| $R^1$ | $R^2$ | $R^3$ | 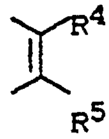 | $R^6$ |
|---|---|---|---|---|
| H | $(CH_3)_3C-$ | H | 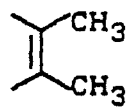 | $-O-SO_2-N(CH_3)_2$ |
| H | $(CH_3)_3C-$ | H | 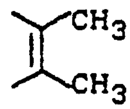 | $-O-SO_2-CH_3$ |
| H | $(CH_3)_3C-$ | H | 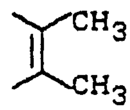 | 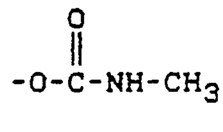 |
| H | $(CH_3)_3C-$ | H | 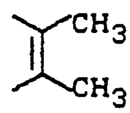 | 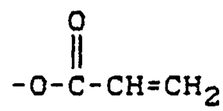 |
| H | $(CH_3)_3C-$ | H | 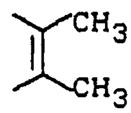 | 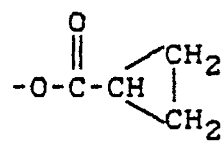 |
| H | $(CH_3)_3C-$ | H | 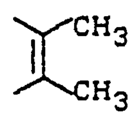 | 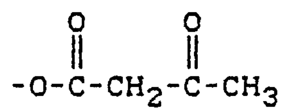 |
| H | $(CH_3)_3C-$ | H | 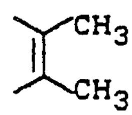 | -SH |
| H | $(CH_3)_3C-$ | H | 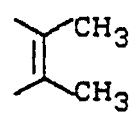 | 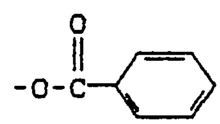 |
| H | $(CH_3)_3C-$ | H | 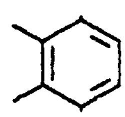 | -OH |
| H | $(CH_3)_3C-$ | H | 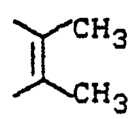 | -OH |

| $R^1$ | $R^2$ | $R^3$ | 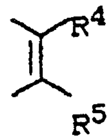 | $R^6$ |
|---|---|---|---|---|
| H | $(CH_3)_3C-$ | H | 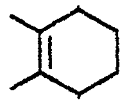 | -OH |
| H | 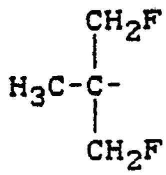 | H | 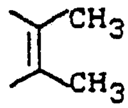 | -OH |
| H | 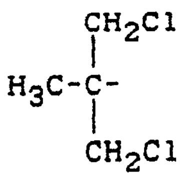 | H | 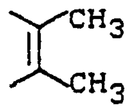 | -OH |
| H | 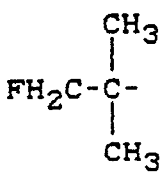 | H | 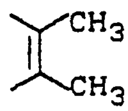 | -OH |
| H | 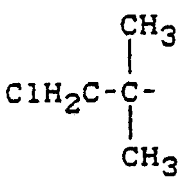 | H | 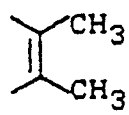 | -OH |
| H | 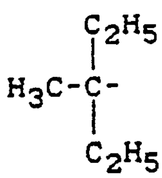 | H | 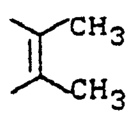 | -OH |
| H | $(CH_3)_2CH-$ | H | 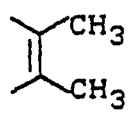 | -OH |
| H | 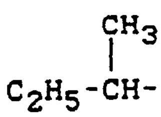 | H | 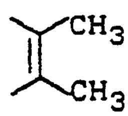 | -OH |

| $R^1$ | $R^2$ | $R^3$ | 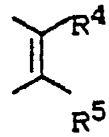 | $R^6$ |
|---|---|---|---|---|
| H | 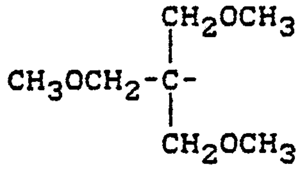 | H | 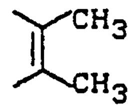 | -OH |
| H | 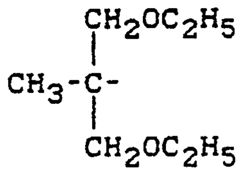 | H | 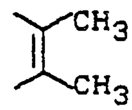 | -OH |
| H | 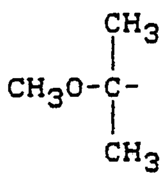 | H | 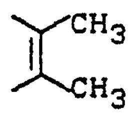 | -OH |
| H | 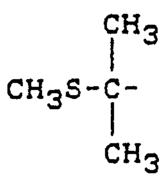 | H | 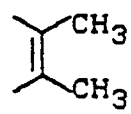 | -OH |
| H | 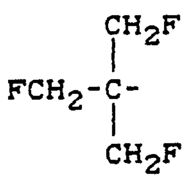 | H | 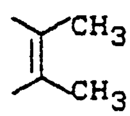 | -OH |
| H | 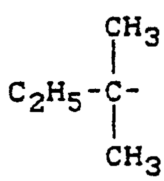 | H | 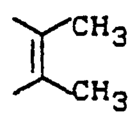 | -OH |

| $R^1$ | $R^2$ | $R^3$ | 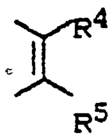 | $R^6$ |
|---|---|---|---|---|
| H | 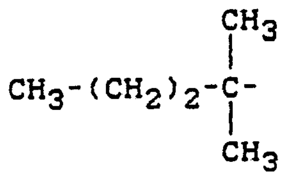 | H | 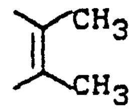 | -OH |
| H | 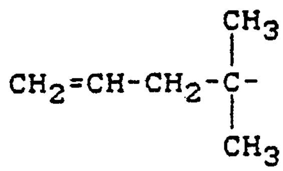 | H | 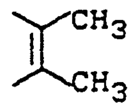 | -OH |
| H | 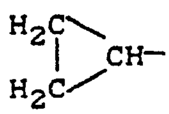 | H | 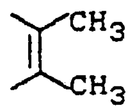 | -OH |
| H | 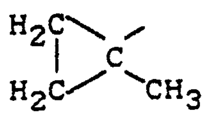 | H | 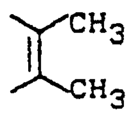 | -OH |
| H | 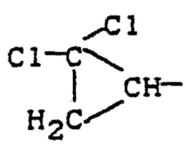 | H | 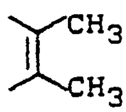 | -OH |
| H | 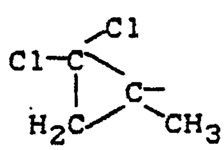 | H | 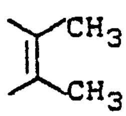 | -OH |

| $R^1$ | $R^2$ | $R^3$ | 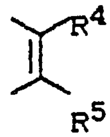 | $R^6$ |
|---|---|---|---|---|
| $CH_3$ | $CH_3$ | H | 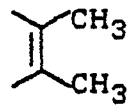 | -OH |
| $CH_3$ | $CH_3$ | H | 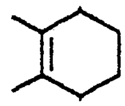 | -OH |
| $CH_3$ | $CH_3$ | H | 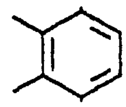 | -OH |
| $(CH_3)_3C-$ | H | H | 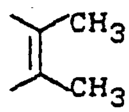 | -OH |
| $(CH_3)_3C-$ | H | H | 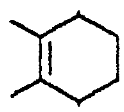 | -OH |
| $(CH_3)_3C-$ | H | H | 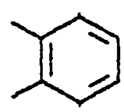 | -OH |
| 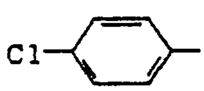 | $CH_3$ | H | 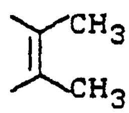 | -OH |
| 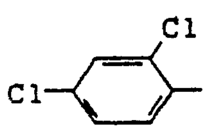 | H | H | 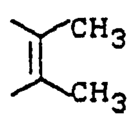 | -OH |
| 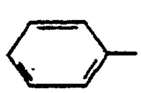 | $CH_3$ | H | 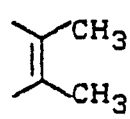 | -OH |
| 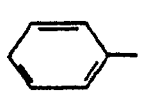 | H | H | 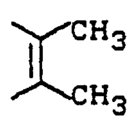 | -OH |

| $R^1$ | $R^2$ | $R^3$ | 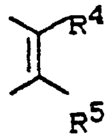 | $R^6$ |
|---|---|---|---|---|
| $CH_2=CH-CH_2-$ | $CH_3$ | H | 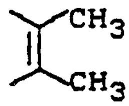 | -OH |
| $HC\equiv C-CH_2-$ | H | H | 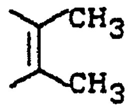 | -OH |
| $Cl-CH=CH-CH_2-$ | $CH_3$ | H | 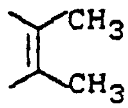 | -OH |
| $Cl-CH=CH-CH_2-$ | $(CH_3)_3C-$ | H | 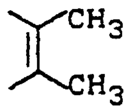 | -OH |
| $C_2H_5O-CH_2CH_2-$ | H | H | 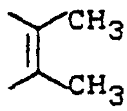 | -OH |
| $C_2H_5O-CH_2CH_2-$ | $CH_3$ | H | 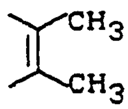 | -OH |
| 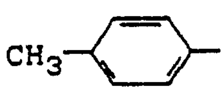 | $CH_3$ | H | 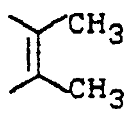 | -OH |
| 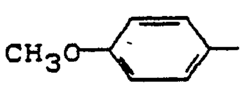 | $CH_3$ | H | 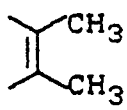 | -OH |
| 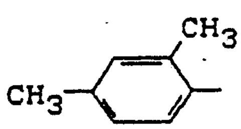 | $CH_3$ | H | 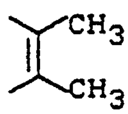 | -OH |
| $F_2CH-$ | $(CH_3)_3C-$ | H | 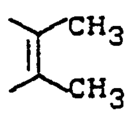 | -OH |

| $R^1$ | $R^2$ | $R^3$ | 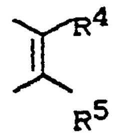 | $R^6$ |
|---|---|---|---|---|
| $F_2CH-$ | H | H | 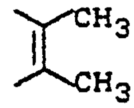 | -OH |
| $CH_3$ | $(CH_3)_3C-$ | H | 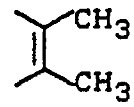 | -OH |
| H | 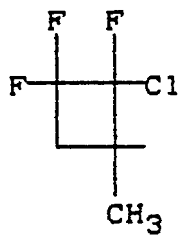 | H | 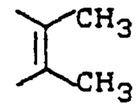 | -OH |

Verwendet man beispielsweise 1-(5-t-Butylpyrazol-3-yl)-3,4-dimethyl-$\Delta^3$-pyrrolin-2,5-dion als Ausgangsverbindung und Natriumborhydrid als Reduktionsmittel, so läßt sich der Reaktionsablauf des erfindungsgemäßen Verfahrens (a) durch das folgende Formelschema darstellen:

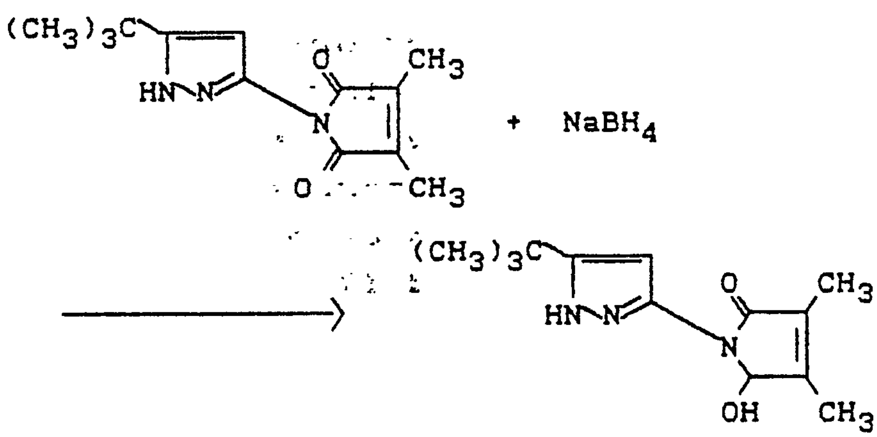


Verwendet man beispielsweise 1-[1-(3,4-Dichlorphenyl)-pyrazol-3-yl]-3,4-dimethyl-$\Delta^3$-pyrrolin-2-ol-5-on und Thionylchlorid als Ausgangsstoffe, so läßt sich der Reaktionsablauf des erfindungsgemäßen Verfahrens (b) durch das folgende Formelschema darstellen:

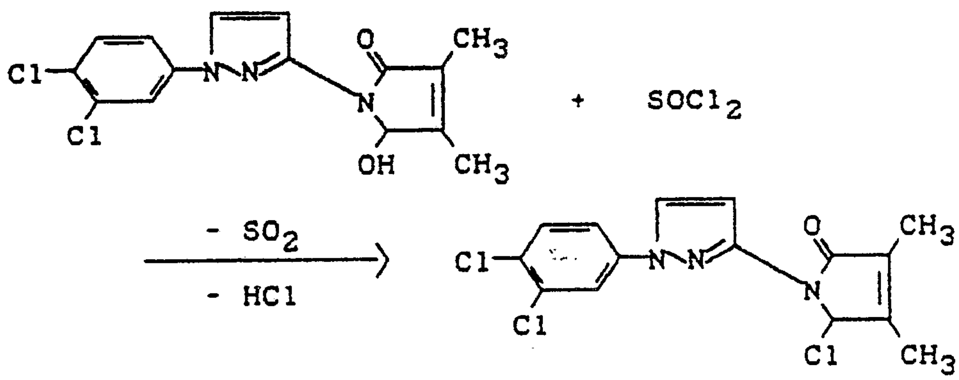


Verwendet man beispielsweise 1-[1-Methyl-5-phenylpyrazol-3-yl]-3,4-dimethyl-$\Delta^3$-pyrrolin-2-ol-5-on und Acetylchlorid als Ausgangsstoffe, so läßt sich der Reaktionsablauf des erfindungsgemäßen Verfahren (c-α) durch das folgende Formelschema darstellen:

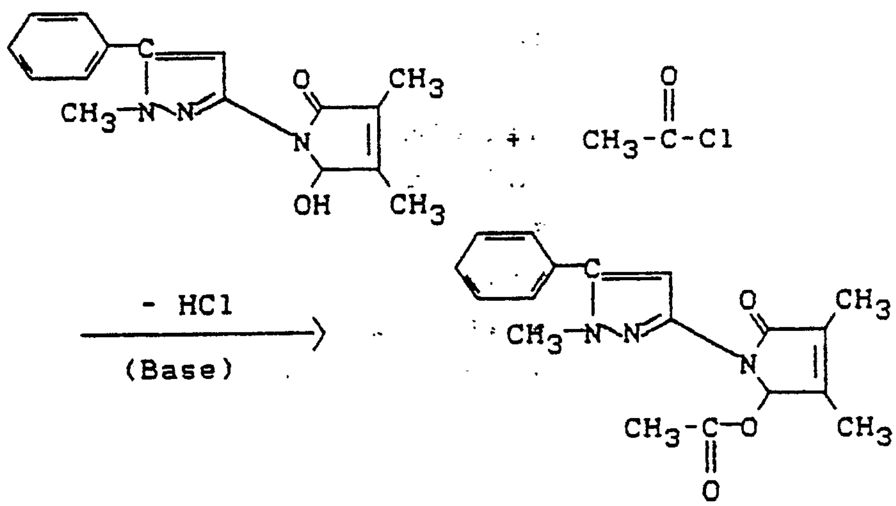


Verwendet man beispielsweise 1-(5-t-Butyl-pyrazol-3-yl)-$\Delta^3$-pyrrolin-2-ol-5-on und Methylisocyanat als Ausgangsstoffe, so läßt sich der Reaktionsablauf des erfindungsgemäßen Verfahrens (c-ß) durch das folgende Formelschema darstellen:

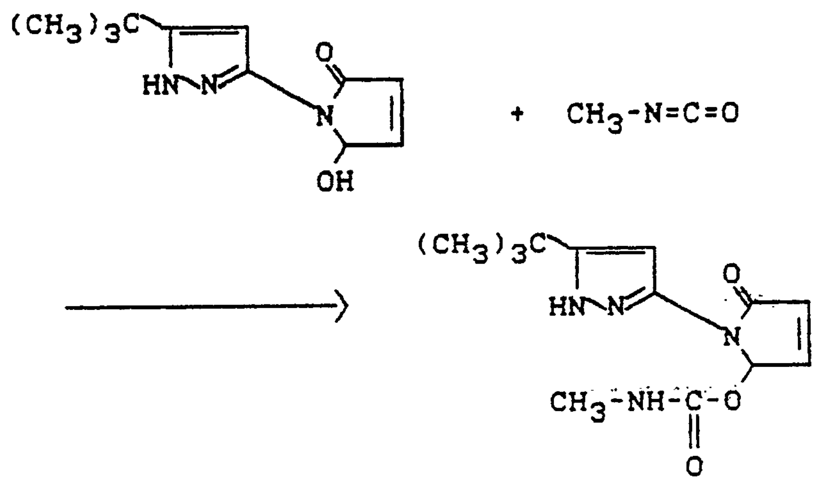

Verwendet man beispielsweise 1-(1,5-Dimethylpyrazol-3-yl)-3,4-dimethyl-$\Delta^3$-pyrrolin-2-ol-5-on und Trifluormethansulfonsäurechlorid als Ausgangsstoffe, so läßt sich der Reaktionsablauf des erfindungsgemäßen

Verfahrens (c-γ) durch das folgende Formelschema darstellen:

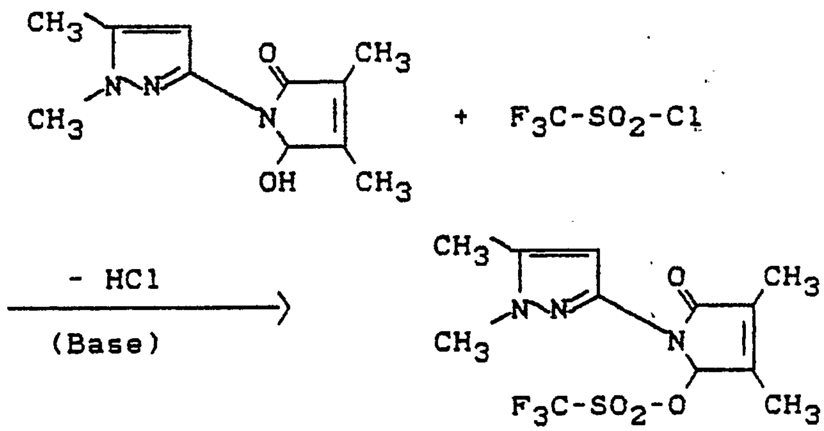


Die zur Durchführung des erfindungsgemäßen Verfahrens (a) als Ausgangsstoffe benötigten Pyrazolylpyrrolindione sind durch die Formel (II) allgemein definiert. In dieser Formel (II) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die Pyrazolylpyrrolindione der Formel (II) sind teilweise bekannt (vergl. z.B. J.chem.Soc. Perkin Trans. I, 1976, 309-312; J. chem. Res.; Synop. 1979, 198 bzw. CA 92: 6458b oder Gazz. chim. Ital. 87, 597-614 [1957] bzw. CA 54: 2333c).

Noch nicht bekannt sind Verbindungen der Formel (IIa),

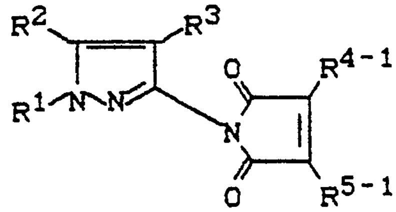
(IIa)

in welcher
$R^1$, $R^2$ und $R^3$ die oben angegebene Bedeutung haben und
$R^{4-1}$ und $R^{5-1}$ entweder unabhängig voneinander jeweils für Wasserstoff oder Alkyl stehen oder gemeinsam für einen zweifach verknüpften Alkandiylrest stehen.

Man erhält sie in Analogie zu bekannten Verfahren (vergl. z.B. DE-OS 27 35 841), beispielsweise wenn man Aminopyrazole der Formel (VII),

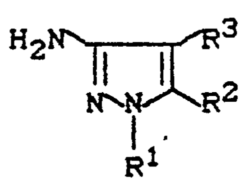
(VII)

in welcher
$R^1$, $R^2$ und $R^3$ die oben angegebene Bedeutung haben,
mit 1,2-Dicarbonsäureanhydriden der Formel (VIII),

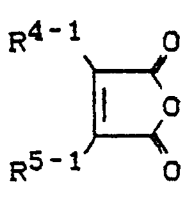
(VIII)

in welcher
$R^{4-1}$ und $R^{5-1}$ die oben angegebene Bedeutung haben,
gegebenenfalls in Gegenwart eines Verdünnungsmittels wie beispielsweise Eisessig und gegebenenfalls in Gegenwart eines Acylierungskatalysators wie beispielsweise 4-(N,N-Dimethylamino)-pyridin bei Temperaturen zwischen 20 °C und 150 °C umsetzt und gegebenenfalls anschließend so erhältliche Verbindungen der Formel (IIb),

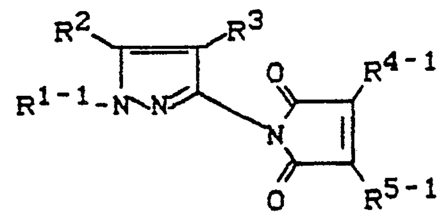

(IIb)

in welcher
$R^2$, $R^3$, $R^{4-1}$ und $R^{5-1}$ die oben angegebene Bedeutung haben und
$R^{1-1}$ für Wasserstoff steht,
am $N^1$-Stickstoffatom des Pyrazolringes in allgemein üblicher Art und Weise mit Alkylierungsmitteln, Alkenylierungsmitteln, Arylierungsmitteln oder Aralkylierungsmitteln wie beispielsweise Alkylhalogeniden, Alkenylhalogeniden, Dialkylsulfaten, Aralkylhalogeniden oder Arylhalogeniden, wobei die Reste Alkyl, Alkenyl, Aralkyl und Aryl die bei der Beschreibung des Restes $R^1$ bevorzugt bzw. besonders bevorzugt angegebene Bedeutung haben, gegebenenfalls in Gegenwart eines Verdünnungsmittels wie beispielsweise Acetonitril, Dimethylformamid oder Toluol und gegebenenfalls in Gegenwart eines Reaktionshilfsmittels wie beispielsweise Natriumhydrid oder Kaliumcarbonat bei Temperaturen zwischen 0 °C und 120 °C alkyliert oder aryliert.

Aminopyrazole der Formel (VII) sind größtenteils bekannt oder erhältlich in Analogie zu bekannten Verfahren (vergl. z.B. J.Indian chem. Soc. 48, 958 [1971]; J. med. Chem. 22, 1385 [1979]; EP 135 252; J.Heterocycl. Chem. 19, 1267 [1982] sowie die Herstellungsbeispiele). 1,2-Dicarbonsäureanhydride der Formel (VIII) sind ebenfalls bekannt oder erhältlich in Analogie zu bekannten Verfahren (vergl. z.B. Gazz. chim. ital. 57, 300-311 [1927]; JP 44/20736; Can. J. Chem. 46, 3189-96 [1968]).

Die zur Durchführung der erfindungsgemäßen Verfahren (b) und (c) als Ausgangsstoffe benötigten Pyrazolylpyrrolinone sind durch die Formel (Ia) allgemein definiert. In dieser Formel (Ia) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die Pyrazolylpyrrolinone der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten nukleophilen Reagenzien sind durch die Formel (III) allgemein definiert. In dieser Formel (III) steht $R^{6-1}$ vorzugsweise für Fluor, Chlor, Brom, Iod, Mercapto, Amino oder für jeweils geradkettiges oder verzweigtes Alkylamino oder Dialkylamino mit jeweils 1 bis 4 Kohlenstoffatomen. $R^{6-1}$ steht insbesondere für Chlor, Brom, Iod, Mercapto, Amino, Methylamino, Ethylamino, Dimethylamino oder Diethylamino.
A steht vorzugsweise für Wasserstoff oder für ein Natrium- oder Kaliumkation.

Auch andere für nukleophile Substitutionsreaktionen übliche Reagenzien wie beispielsweise Thionylchlorid, Phosphoroxychlorid, Phosphoroxybromid, Thioharnstoff oder N,N-Dimethylthioformamid kommen als Ausgangsverbindungen zur Durchführung des erfindungsgemäßen Verfahrens (b) infrage.
Ganz besonders bevorzugt als nukleophile Reagenzien der Formel (III) sind Thionylchlorid, Natriumiodid, Kaliumhydrogensulfid, Thioharnstoff, Ammoniak, Methylamin, Ethylamin, Dimethylamin oder Diethylamin.
Die nukleophilen Reagenzien der Formel (III) sind allgemein bekannte Verbindungen.
Die zur Durchführung des erfindungsgemäßen Verfahrens (c-α) weiterhin als Ausgangsstoffe benötigten Acylierungsmittel sind durch die Formel (IV) allgemein definiert. In dieser Formel (IV) steht $R^7$ vorzugsweise für diejenigen Reste, die bereits im Zusammenhang mit der Beschreibung der erfindungsgemäßen Stoffe der Formel (I) als bevorzugt für diese Substituenten genannt wurden. $E^1$ steht vorzugsweise für Halogen, insbesondere für Chlor oder Brom oder für einen Anhydridrest der Formel $R^7\text{-}\underset{O}{\underset{\|}{C}}\text{-O-}$,
wobei $R^7$ die oben angegebene Bedeutung hat.

Die zur Durchführung des erfindungsgemäßen Verfahrens (c-β) weiterhin als Ausgangsstoffe benötigten Isocyanate sind durch die Formel (V) allgemein definiert. In dieser Formel (V) steht $R^{7-1}$ vorzugsweise für

gerad kettiges oder verzweigtes Alkyl mit 1 bis 6 Kohlenstoffatomen, insbesondere mit 1 bis 4 Kohlenstoffatomen. $R^{7-1}$ steht ganz besonders bevorzugt für Methyl oder Ethyl.

Die zur Durchführung des erfindungsgemäßen Verfahrens (c-γ) weiterhin als Ausgangsstoffe benötigten Sulfonylierungsmittel sind durch die Formel (VI) allgemein definiert. In dieser Formel (VI) steht $R^8$ vorzugsweise für diejenigen Reste, die bereits im Zusammenhang mit der Beschreibung der erfindungsgemäßen Stoffe der Formel (I) als bevorzugt für diese Substituenten genannt wurden.
$E^2$ steht vorzugsweise für Halogen, insbesondere für Chlor.

Die Acylierungsmittel der Formel (IV), die Isocyanate der Formel (V) und die Sulfonylierungsmittel der Formel (VI) sind allgemein bekannte Verbindungen der organischen Chemie.

Als Reduktionsmittel zur Durchführung des erfindungsgemäßen Verfahrens (a) kommen alle üblichen für derartige Reduktionsreaktionen verwendbaren Reduktionsmittel infrage. Vorzugsweise verwendet man komplexe Hydride, wie beispielsweise Natriumborhydrid, Natriumcyanoborhydrid, Lithiumborhydrid, Lithiumaluminiumhydrid oder Isobutylaluminiumhydrid.

Als Verdünnungsmittel zur Durchführung des erfindungsgemäßen Verfahrens (a) kommen in Abhängigkeit vom verwendeten Reduktionsmittel alle üblichen organischen oder anorganischen Lösungsmittel infrage. Vorzugsweise verwendet man Ether, wie Diethylether, Dioxan oder Tetrahydrofuran oder Alkohole, wie Methanol,. Ethanol oder Propanol.

Die Reaktionstemperaturen können bei der Durchführung des erfindungsgemäßen Verfahrens (a) in Abhängigkeit vom verwendeten Reduktionsmittel in einem größeren Bereich variiert werden. Im allgemeinen arbeitet man bei Temperaturen zwischen - 20 °C und + 150 °C, vorzugsweise bei Temperaturen zwichen 0 °C und 120 °C.

Zur Durchführung des erfindungsgemäßen Verfahrens (a) setzt man pro Mol an Pyrazolylpyrrolindion der Formel (II) im allgemeinen 0.1 bis 2.0 Mol, vorzugsweise 0.25 bis 1.5 Mol an Reduktionsmittel ein.
Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahren (b) kommen organische oder anorganische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Dichlorethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide oder Sulfone, wie Dimethylsulfoxid oder Sulfolan, Basen, wie Pyridin, Alkohole, wie Methanol, Ethanol, Propanol, Butanol, Ethylenglykol oder Ethylenglykolmonomethylether, Wasser oder auch Mischungen von Wasser und einem der oben genannten Lösungsmittel.

Das erfindungsgemäße Verfahren (b) kann gegebenenfalls in Gegenwart eines geeigneten Reaktionshilfsmittels durchgeführt werden. Als solche kommen insbesondere organische Amine oder Amide infrage. Vorzugsweise verwendet man Pyridin, Dimethylanilin oder Dimethylformamid.

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +200 °C, vorzugsweise bei Temperaturen zwischen 0 °C und 150 °C.

Zur Durchführung des erfindungsgemäßen Verfahrens (b) setzt man pro Mol an Pyrazolylpyrrolinon der Formel (Ia) im allgemeinen 1.0 bis 5.0 Mol, vorzugsweise 1.0 bis 2.5 Mol an nukleophilen Reagenz der Formel (III) und gegebenenfalls 1.0 bis 2.0 Mol, vorzugsweise bis 1.5 Mol an Reaktionshilfsmitteln ein.
Die Reaktionsdurchführung, Aufarbeitung und Isolierung der Reaktionsprodukte der Formel (Ib) erfolgt nach allgemein üblichen Methoden.

Dabei kann es gegebenenfalls von Vorteil sein, die nukleophile Substitution gemäß dem erfindungsgemäßen Verfahren (b) mehrmals hintereinander durchzuführen, um bestimmte Substituenten an der Stelle von $R^6$ zu erhalten.
So ist es beispielsweise möglich, die Pyrazolylpyrrolinone der Formel (Ia) zunächst mit Thionylchlorid in die entsprechenden Chlorverbindungen zu überführen und anschließend mit Natriumiodid den Chlorsubstituenten gegen Iod auszutauschen oder die so erhältlichen Iodverbindungen entweder mit Kaliumhydrogensulfid oder Thioharnstoff in die entsprechenden Mercaptoverbindungen oder mit Ammoniak in die entsprechenden Aminoverbindungen zu überführen.
Auch die Durchführung dieser Austauschreaktionen erfolgt in Analogie zu allgemein üblichen Verfahren (vergl. z.B. "Organikum", S.234-275, VEB Deutscher Verlag der Wissenschaften, Berlin 1981).

Zur Durchführung des erfindungsgemäßen Verfahrens (c) kommen als Verdünnungsmittel inerte organische Lösungsmittel infrage. Vorzugsweise verwendet man aliphatische, alicyclische oder aromatische,

gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Essigsäureethylester, Säuren, wie Essigsäure, Nitrile, wie wie Acetonitril oder Propionitril oder Amide, wie Dimethylformamid, Dimethylacetamid, N-Methylpyrrolidon oder Hexamethylphosphorsäuretriamid. Verwendet man als Reaktionspartner in den Varianten (c-α), (c-β) oder (c-γ) Verbindungen der Formeln (IV), (V) oder (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (c) kommen alle üblicherweise verwendbaren anorganischen und organischen Basen in Frage. Vorzugsweise verwendet man Alkalimetallhydroxide, -carbonate oder -hydrogencarbonate, wie beispielsweise Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (c) setzt man pro Mol an Pyrazolylpyrrolinon der Formel (Ia) in den Varianten (c-α), (c-β) oder (c-γ) im allgemeinen jeweils 1,0 bis 20,0 Mol, vorzugsweise jeweils 1,0 bis 15,0 Mol, an Acylierungsmittel der Formel (IV) oder Isocyanat der Formel (V) oder Sulfonylierungsmittel der Formel (VI) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (Ic) erfolgt nach üblichen Methoden.

Die Abtrennung von gegebenenfalls auftretenden Nebenprodukten (beispielsweise können gegebenenfalls, falls der Substituent $R^1$ am Pyrazolring für Wasserstoff steht, in Nebenreaktion auch Acylierungen am Pyrazol-Stickstoff stattfinden) erfolgt ebenfalls mit Hilfe allgemein üblicher Trennmethoden (beispielsweise durch Säulenchromatographie).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfen anlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung von dikotylen Unkräutern in mono- und dikotylen Kulturen, wie beispielsweise Soja oder Weizen einsetzen.

Aus die Zwischenprodukte der Formel (II) besitzen gute herbizide Wirksamkeit.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Darüberhinaus zeigen die erfindungsgemäßen Wirkstoffe in geeigneten Aufwandmengen auch insektizide Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on zur (METAMITRON) Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 2,4-Dichlorphenoxypropionsäure (2,4-DP); 4-(2,4-Dichlorphenoxy)-buttersäure (2,4-DB); (2-Methyl-4-chlorphenoxy)-essigsäure (MCPA); (4-Chlor-2-methylphenoxy)-propionsäure (MCPP); [(4-Amino-3,5-dichlor-6-fluor-2-pyridinyl)-oxy]-essigsäure bzw. deren 1-Methylheptylester (FLUROXYPYR); 2-[4-(2,4-Dichlorphenoxy)-phenoxy]-propionsäure, deren Methyl- oder deren Ethylester (DICLOFOP); 2-{4-[(6-Chlor-2-benzoxazolyl)-oxy]-phenoxy}-propansäure, deren Methyl-oder deren Ethylester (FENOXAPROP); Chloressigsäure-N-(methoxymethyl)-2,6-diethylanilid (ALACHLOR); 2-Ethyl-6-methyl-N-(1-methyl-2-methoxyethyl)-chloracetanilid (METOLACHLOR); N-(1-Ethylpropyl)-3,4-dimethyl-2,6-dinitroanilin (PENDIMETHALIN); 2,6-Dinitro-4-trifluormethyl-N,N-dipropylanilin (TRIFLURALIN); 5-(2-Chlor-4-trifluormethylphenoxy)-N-methylsulfonyl-2-nitrobenzamid (FOMESAFEN); N,N-Dimethyl-N′-(3-chlor-4-methylphenyl)-harnstoff (CHLORTOLURON); N,N-Dimethyl-N′-(4-isopropylphenyl)-harnstoff (ISOPROTURON); 2-[4,5-Dihydro-4-methyl-4-isopropyl-5-oxo-(1H)-imidazol-2-yl]-5 ethyl-pyridin-3-carbonsäure (IMAZETHAPYR); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 3,5-Dibrom-4-hydroxy-benzonitril (BROMOXYNIL); 3,5-Diiod-4-hydroxybenzonitril (IOXYNIL); N-Methyl-2-(1,3-benzthiazol-2-yloxy)-acetanilid (MEFENACET); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); 2-{[[((4-Methoxy-6-methyl-1,3,5-triazin-2-yl)-amino)-carbonyl]-amino]-sulfonyl}-benzoesäure oder deren Methylester (METSULFURON); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2-Chlor-4-ethylamino-6-isopropylamino-1,3,5-triazin (ATRAZIN); 2-Chlor-4-ethylamino-6-(3-cyanopropylamino)-1,3,5-triazin (CYANAZIN); 4-Ethylamino-2-t-butylamino-6-methylthio-s-triazin (TERBUTRYNE); 3-Isopropyl-2,1,3-benzothiadiazin-4-on-2,2-dioxid (BENTAZON); 4-Amino-6-t-butyl-3-ethylthio-1,2,4-triazin-5(4H)-on (ETHIOZIN); 2-[(2-Chlorphenyl)-methyl]-4,4-dimethylisoxazolidin-3-on (DIMETHAZONE) und O-(6-Chlor-3-phenyl-pyridazin-4-yl)-S-octyl-thiocarbonat (PYRIDATE) sind möglich. Einige Mischungen zeigen überraschenderweise auch synergistische Wirkung.

Auch eine Mischung mit anderen bekannten Wirkstoffen, wie Fungizide, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und Verwendung der erfindungsgemäßen Wirkstoffe geht aus den nachfolgenden Beispielen hervor.

<u>Herstellungsbeispiele:</u>

Beispiel 1:

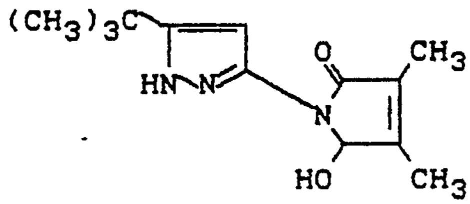

(Verfahren a)

Zu 29,6 g (0,12 Mol) 1-(5-t-Butylpyrazol-3-yl)-3,4-dimethyl-$\Delta^3$-pyrrolin-2,5-dion in 500 ml Methanol gibt man unter Rühren und Kühlung 4,6 g (0,12 Mol) Natriumborhydrid, so daß die Temperatur der Reaktionsmischung 35 °C nicht übersteigt, rührt anschließend 5 Stunden bei Raumtemperatur, engt dann im Vakuum ein, nimmt den Rückstand in Dichlormethan auf, wäscht mit Wasser, trocknet über Natriumsulfat und entfernt das Lösungsmittel im Vakuum.

Man erhält 24 g (80 % der Theorie) an 1-(5-t-Butylpyrazol-3-yl)-3,4-dimethyl-$\Delta^3$-pyrrolin-2-ol-5-on vom Schmelzpunkt 207 °C.

Beispiel 2:

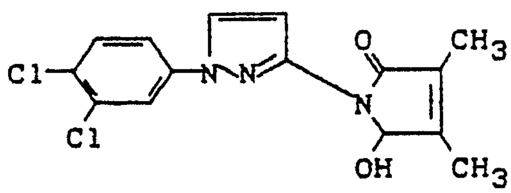

(Verfahren a)

Zu 3,4 g (0,01 Mol) 1-[1-(3,4-Dichlorphenyl)-pyrazol-3-yl]-3,4-dimethyl-$\Delta^3$-pyrrolin-2,5-dion in 55 ml Methanol gibt man 0,6 g (0,016 Mol) Natriumborhydrid, rührt 2 Stunden bei Raumtemperatur, gibt dann die Reaktionsmischung in 200 ml Wasser, filtriert den ausgefallenen Niederschlag ab, wäscht mit Wasser und trocknet.

Man erhält 3,2 g (94 % der Theorie) an 1-[1-(3,4-Dichlorphenyl)-pyrazol-3-yl]-3,4-dimethyl-$\Delta^3$-pyrrolin-2-ol-5-on vom Schmelzpunkt 188 °C.

In entsprechender Weise erhält man die folgenden Pyrazolylpyrrolinone der allgemeinen Formel (I):

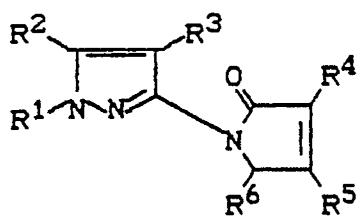

(I)

Tabelle 1

| Bsp. Nr. | $R^1$ | $R^2$ | $R^3$ | 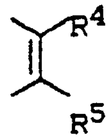 | $R^6$ | Schmelzpunkt/°C |
|---|---|---|---|---|---|---|
| 3 | H | H | H | 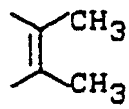 | OH | 120-123 |
| 4 | H | $CH_3$ | H | 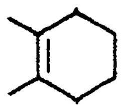 | OH | 219 |
| 5 | H | $CH_3$ | H | 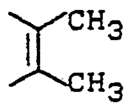 | OH | 192 |
| 6 | H | 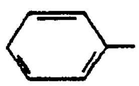 | H | 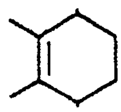 | OH | 196-200 (Zers.) |
| 7 | H | 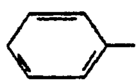 | H | 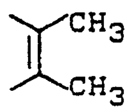 | OH | 204 |
| 8 | H | $(CH_3)_3C-$ | H | 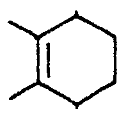 | OH | 203 |
| 9 | H | $C(CH_3)_3$ | H | 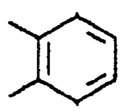 | OH | 95 |
| 10 | $CH_3$ | $CH_3$ | H | 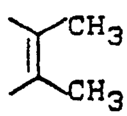 | OH | 161 |
| 11 | H | 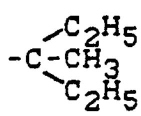 | H | 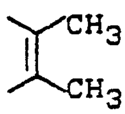 | OH | 188 |

Tabelle 1

| Bsp. Nr. | $R^1$ | $R^2$ | $R^3$ | 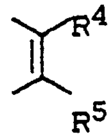 | $R^6$ | Schmelzpunkt/°C |
|---|---|---|---|---|---|---|
| 12 | H | 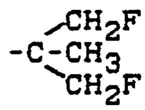 | H | 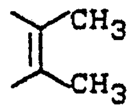 | OH | 175 |
| 13 | H | $-C(CH_3)_3$ | $CH_3$ | 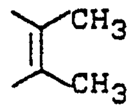 | OH | 94 |
| 14 | H | 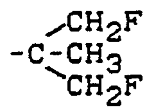 | H | 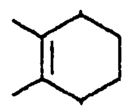 | OH | 208 |
| 15 | H | 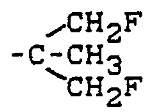 | H | 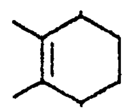 | OH | 185 |

Herstellung der Ausgangsverbindungen:

Beispiel IIa-1:

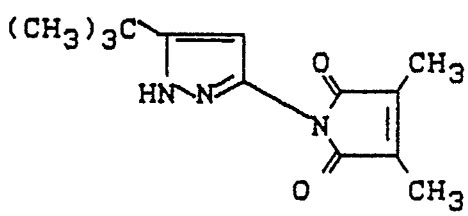

9,5 g (0,075 Mol) Dimethylmaleinsäureanhydrid und 10,4 g (0,075 Mol) 3-Amino-5-t-butylpyrazol werden in 150 ml Eisessig 16 Stunden bei Rückflußtemperatur gerührt, abgekühlt, im Vakuum eingeengt, der Rückstand mit Petrolether verrührt, abgesaugt und aus Isopropanol umkristallisiert.

Man erhält 11,0 g (59 % der Theorie) an 1-(5-t-Butylpyrazol-3-yl)-3,4-dimethyl-$\Delta^3$-pyrrolin-2,5-dion vom Schmelzpunkt 168° C.

Beispiel IIa-2:

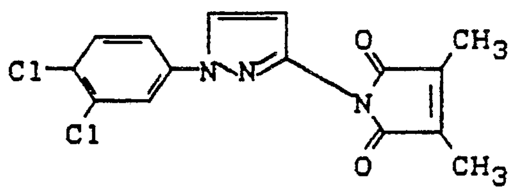

5,0 g (0,02 Mol) 3-Amino-1-(3,4-dichlorphenyl)-pyrazol (vergl. US-PS 4 149 005) und 2,3 g (0,023 Mol) Dimethylmaleinsäureanhydrid werden in 30 ml Eisessig 2 Stunden bei 60° C gerührt. Zur Aufarbeitung wird die Reaktionsmischung in 300 ml Wasser gegeben, der kristalline Niederschlag abfiltriert, mit Wasser gewaschen und getrocknet.

Man erhält 6,4 g (87 % der Theorie) an 1-[1-(3,4-Dichlorphenyl)-pyrazol-3-yl]-3,4-dimethyl-$\Delta^3$-pyrrolin-2,5-dion vom Schmelzpunkt 147° C.

In entsprechender Weise und gemäß den allgemeinen Angaben zur Herstellung erhält man die folgenden Pyrazolylpyrrolindione der allgemeinen Formel (IIa):

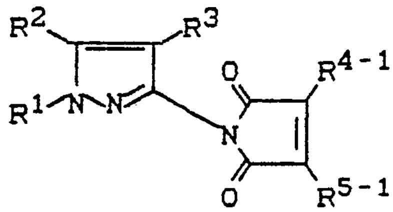

(IIa)

Tabelle 2

| Bsp. Nr. | $R^1$ | $R^2$ | $R^3$ | 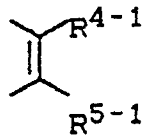 | Schmelzpunkt/° C |
|---|---|---|---|---|---|
| IIa-3 | H | H | H | 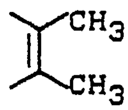 | 153-155 |
| IIa-4 | H | $CH_3$ | H | 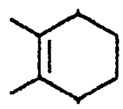 | 225-226 |
| IIa-5 | H | $CH_3$ | H | 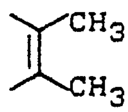 | 222-223 |
| IIa-6 | H | 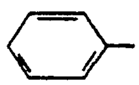 | H | 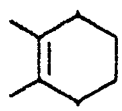 | >250 |
| IIa-7 | H | 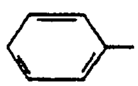 | H | 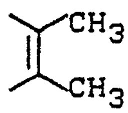 | >250 |
| IIa-8 | H | $(CH_3)_3C-$ | H | 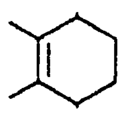 | 220 |
| IIa-9 | H | $-C(CH_3)_3$ | H | 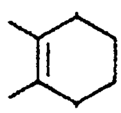 | 139-140 |
| IIa-10 | H | 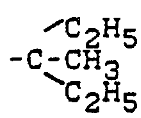 | H | 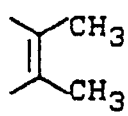 | 163 |
| IIa-11 | H | 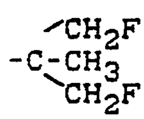 | H | 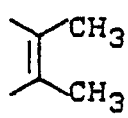 | 129 |

Tabelle 2

| Bsp. Nr. | $R^1$ | $R^2$ | $R^3$ | 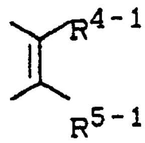 | Schmelz-punkt/° C |
|---|---|---|---|---|---|
| IIa-12 | H | $-C(CH_3)_3$ | $CH_3$ | 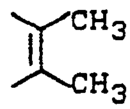 | 193 |
| IIa-13 | H | 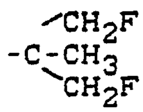 | H | 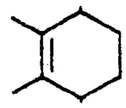 | 188 |
| IIa-14 | H | 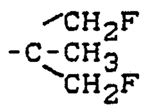 | H | 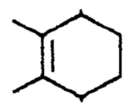 | 170 |

Beispiel VII-1:

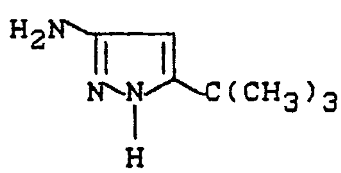

62,5 g (0,5 Mol) 1-Cyano-3,3-dimethylbutan-2-on und 25 g (0,5 Mol) Hydrazinhydrat werden in 500 ml Ethanol 16 Stunden bei Rückflußtemperatur gerührt, abgekühlt auf 60° C, mit 1 ml konzentrierter Schwefelsäure versetzt und weitere 4 Stunden gerührt, wobei die Mischung auf Raumtemperatur abkühlt. Zur Aufarbeitung wird im Vakuum eingeengt, der Rückstand in Dichlormethan aufgenommen, zweimal mit jeweils 100 ml Wasser und zweimal mit jeweils 100 ml gesättigter wässriger Natriumhydrogencarbonatlösung gewaschen, über Natriumsulfat getrocknet und im Vakuum eingeengt.

Man erhält 64 g (92 % der Theorie) an 3(5)-Amino-5(3)-t-butylpyrazol als Öl.

$^1H$-NMR ($CDCl_3$/Tetramethylsilan): $\delta$ = 1.29ppm (s, t-Bu)

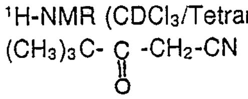

Zu einer Lösung aus 201,8 g (1,5 Mol) 1-Chlor-3,3-dimethyl-2-butanon und 800 ml Methanol tropft man bei Siedetemperatur eine Lösung aus 108,1 g (1,66 Mol) Kaliumcyanid in 270 ml Wasser und erhitzt 1 Stunde unter Rückfluß. Anschließend wird das Lösungsmittel abgezogen, der Rückstand in 1,5 l Eiswasser angeschlämmt, durch Zugabe von 66,4 g (1,5 Mol) Natriumhydroxid in Lösung gebracht und mit Ether gewaschen. Die wässrige Phase wird unter Eiskühlung angesäuert, mit Methylenchlorid extrahiert, über Magnesiumsulfat getrocknet und eingeengt.

Nach Umkristallisation aus Essigester/Ligroin erhält man 139,6 g (74,5 % der Theorie) an 1-Cyano-3,3-dimethylbutan-2-on vom Schmelzpunkt 67-69° C.

Analog Beispiel VII-1 erhält man die folgenden Aminopyrazole der Formel (VII):

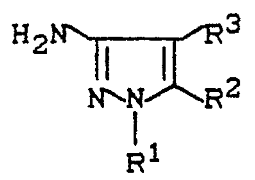

(VII)

Tabelle 3

| Bsp.Nr | $R^1$ | $R^2$ | $R^3$ | physikalische Eigenschaften |
|---|---|---|---|---|
| VII-2 | H | $CH_3$ | H | Kp: 155° C/3mbar |
| VII-3 | H | 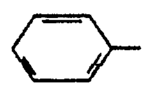 | H | Fp: 116° C |

Anwendungsbeispiele:

In den folgenden Anwendungsbeispielen wurde die nachstehend aufgeführte Verbindung als Vergleichssubstanz eingesetzt:

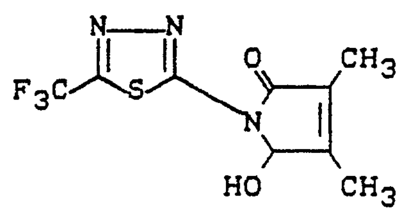

(A)

3,4-Dimethyl-5-hydroxy-1-(5-trifluormethyl-1,3,4-thiadiazol-2-yl)-$\Delta^3$-pyrrolin-2-on (bekannt aus DE-OS 27 35 841)

Beispiel A

Pre-emergence-Test

| | | |
|---|---|---|
| Lösungsmittel: | 5 Gewichtsteile | Aceton |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in %

Schädigung im Vergleich zur Entwicklung der unbehandelten Kontrolle. Es bedeuten:

| | | |
|---|---|---|
| 0 % | = | keine Wirkung (wie unbehandelte Kontrolle) |
| 100 % | = | totale Vernichtung |

Eine deutliche Überlegenheit in der Wirksamkeit ebenso wie in der Nutzpflanzenselektivität gegenüber der Vergleichssubstanz (A) zeigen in diesem Test z.B. die Verbindungen gemäß den Herstellungsbeispielen 1 und 8.

Beispiel B

Post-emergence-Test

| | | |
|---|---|---|
| Lösungsmittel: | 5 Gewichtsteile | Aceton |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

| | | |
|---|---|---|
| 0 % | = | keine Wirkung (wie unbehandelte Kontrolle) |
| 100 % | = | totale Vernichtung |

Eine deutliche Überlegenheit in der Wirksamkeit bei vergleichbarer Nutzpflanzenselektivität gegenüber der Vergleichssubstanz (A) zeigen in diesem Test z.B. die Verbindungen gemäß folgender Herstellungsbeispiele: 1, 5 und 8.

## Ansprüche

1. Pyrazolylpyrrolinone der Formel (I),

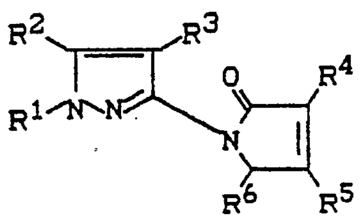

(I)

in welcher

$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,

$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,

$R^3$ für Wasserstoff oder Alkyl steht,

$R^4$ und $R^5$ entweder unabhängig voneinander jeweils für Wasserstoff oder Alkyl stehen oder gemeinsam für einen jeweils zweifach verknüpften Alkandiyl-, Alkendiyl- oder Alkdiendiylrest stehen und
$R^6$ für Halogen, Hydroxy, Mercapto, Amino, Alkylamino, Dialkylamino, für einen Rest $R^7-\underset{\overset{\|}{O}}{C}-O$ oder für einen Rest $R^8-SO_2-O-$ steht,
wobei
$R^7$ für Wasserstoff, Alkyl, Alkenyl, Alkinyl, Halogenalkyl, Alkoxyalkyl, Alkylthioalkyl, Alkylcarbonylalkyl, Alkoxycarbonylalkyl, für Alkylamino, für Dialkylamino oder für jeweils gegebenenfalls substituiertes Cycloalkyl oder Aryl steht und
$R^8$ für Alkyl, Halogenalkyl, Alkylamino, Dialkylamino oder für gegebenenfalls substituiertes Aryl steht.

2. Pyrazolylpyrrolinone der Formel (I) gemäß Anspruch 1, in welcher
$R^1$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 6 Kohlenstoffatomen und 1 bis 13 gleichen oder verschiedenen Halogenatomen, Halogenalkenyl mit 3 bis 6 Kohlenstoffatomen und 1 bis 11 gleichen oder verschiedenen Halogenatomen oder Alkoxyalkyl mit jeweils 1 bis 4 Kohlenstoffatomen in den einzelnen Alkylteilen oder für jeweils gegebenenfalls einfach oder mehrfach, gleich oder verschieden substituiertes Benzyl, Phenylethyl oder Phenyl steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen sowie jeweils geradkettiges oder verzweigtes Alkoxycarbonyl oder Alkoximinoalkyl mit jeweils 1 bis 4 Kohlenstoffatomen in den einzelnen Alkylteilen;
$R^2$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Alkoxyalkyl oder Alkylthioalkyl mit jeweils 1 bis 4 Kohlenstoffatomen in den einzelnen Alkylteilen und 1 bis 3 Alkoxy- bzw. Alkylthiogruppen oder für gegebenenfalls einfach oder mehrfach, gleich oder verschieden substituiertes Cycloalkyl mit 3 bis 7 Kohlenstoffatomen steht, wobei als Substituenten jeweils infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen; oder $R^2$ für gegebenenfalls einfach oder mehrfach, gleich oder verschieden substituiertes Phenyl steht, wobei als Substituenten die bei $R^1$ genannten infrage kommen;
$R^3$ für Wasserstoff oder für geradkettiges oder verzweigtes Alkyl mit 1 bis 4 Kohlenstoffatomen steht,
$R^4$ und $R^5$ entweder unabhängig voneinander jeweils für Wasserstoff oder für geradkettiges oder verzweigtes Alkyl mit 1 bis 4 Kohlenstoffatomen stehen oder gemeinsam für einen jeweils zweifach verknüpften Butan-1,4-diylrest, Buten-1,4-diylrest oder Butadien-1,4-diylrest stehen und
$R^6$ für Fluor, Chlor, Brom, Iod, Hydroxy, Mercapto, Amino, für jeweils geradkettiges oder verzweigtes Alkylamino oder Dialkylamino mit jeweils 1 bis 4 Kohlenstoffatomen in den einzelnen Alkylteilen, für einen Rest $R^7-\underset{\overset{\|}{O}}{C}-O-$ oder für einen Rest $R^8-SO_2-O-$ steht, wobei
$R^7$ für Wasserstoff, für jeweils geradkettiges oder verzweigtes Alkyl mit 1 bis 6 Kohlenstoffatomen, Alkenyl mit 2 bis 6 Kohlenstoffatomen, Alkinyl mit 2 bis 6 Kohlenstoffatomen, Halogenalkyl mit 1 bis 6 Kohlenstoffatomen und 1 bis 13 gleichen oder verschiedenen Halogenatomen, Alkoxyalkyl, Alkylthioalkyl, Alkylcarbonylalkyl oder Alkoxycarbonylalkyl mit jeweils 1 bis 4 Kohlenstoffatomen in den einzelnen Alkylteilen, Alkylamino oder Dialkylamino mit jeweils 1 bis 4 Kohlenstoffatomen in den einzelnen Alkylteilen oder für gegebenenfalls einfach oder mehrfach, gleich oder verschieden substituiertes Cycloalkyl mit 3 bis 7 Kohlenstoffatomen steht, wobei als Substituenten jeweils infrage kommen:
Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen; oder für gegebenenfalls einfach oder mehrfach, gleich oder verschieden substituiertes Phenyl steht, wobei als Substituenten die bei $R^1$ genannten infrage kommen und
$R^8$ für jeweils geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, Alkylamino oder Dialkylamino mit jeweils 1 bis 4 Kohlenstoffatomen in den einzelnen Alkylteilen oder für gegebenenfalls einfach oder mehrfach, gleich oder verschieden substituiertes Phenyl steht, wobei als Substituenten die bei $R^1$ genannten infrage kommen.

3. Verfahren zur Herstellung der Pyrazolylpyrrolinone der allgemeinen Formel (I),

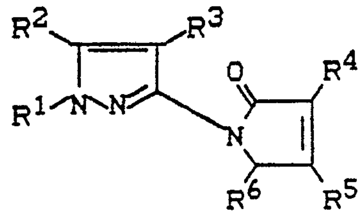

(I)

in welcher

$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,

$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,

$R^3$ für Wasserstoff oder Alkyl steht,

$R^4$ und $R^5$ entweder unabhängig voneinander jeweils für Wasserstoff oder Alkyl stehen oder gemeinsam für einen jeweils zweifach verknüpften Alkandiyl-, Alkendiyl- oder Alkdiendiylrest stehen und

$R^6$ für Halogen, Hydroxy, Mercapto, Amino, Alkylamino, Dialkylamino, für einen Rest $R^7-\underset{\underset{O}{\|}}{C}-O$ oder für einen Rest $R^8-SO_2-O-$ steht,

wobei

$R^7$ für Wasserstoff, Alkyl, Alkenyl, Alkinyl, Halogenalkyl, Alkoxyalkyl, Alkylthioalkyl, Alkylcarbonylalkyl, Alkoxycarbonylalkyl, für Alkylamino, für Dialkylamino oder für jeweils gegebenenfalls substituiertes Cycloalkyl oder Aryl steht und

$R^8$ für Alkyl, Halogenalkyl, Alkylamino, Dialkylamino oder für gegebenenfalls substituiertes Aryl steht,

dadurch gekennzeichnet, daß man

(a) Pyrazolylpyrrolinone der Formel (Ia),

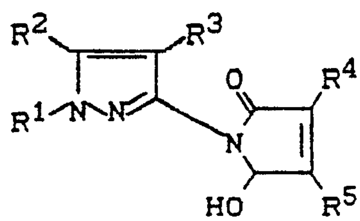

(Ia)

in welcher

$R^1$, $R^2$, $R^3$, $R^4$ und $R^5$ die oben angegebene Bedeutung haben,

erhält, wenn man Pyrazolylpyrrolindione der Formel (II),

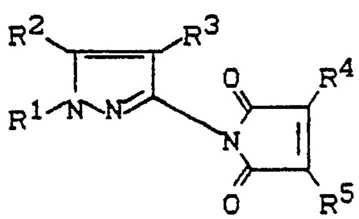

(II)

in welcher

$R^1$, $R^2$, $R^3$, $R^4$ und $R^5$ die oben angegebene Bedeutung haben,

mit einem Reduktionsmittel gegebenenfalls in Gegenwart eines Verdünnungsmittels umsetzt;

oder daß man

(b) Pyrazolylpyrrolinone der Formel (Ib),

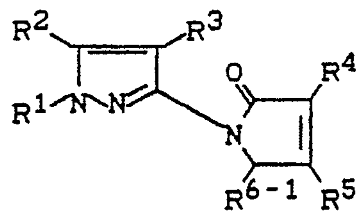

(Ib)

in welcher
$R^{6-1}$ für Halogen, Mercapto, Amino, Alkylamino oder Dialkylamino steht und
$R^1$, $R^2$, $R^3$, $R^4$ und $R^5$ die oben angegebene Bedeutung haben,
erhält, wenn man die mit Hilfe des erfindungsgemäßen Verfahrens (a) erhältlichen Pyrazolylpyrrolinone der Formel (Ia),

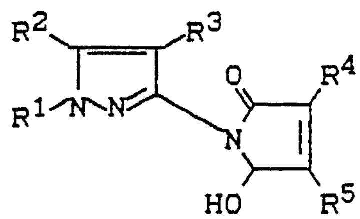

(Ia)

in welcher
$R^1$, $R^2$, $R^3$, $R^4$ und $R^5$ die oben angegebene Bedeutung haben,
mit nukleophilen Reagenzien der Formel (III),
$R^{6-1}$-A (III)
in welcher
A für Wasserstoff, für ein Alkalimetallkation oder für einen anderen geeigneten Abgangsrest steht und
$R^{6-1}$ die oben angegebene Bedeutung hat,
gegebenenfalls in Gegenwart eines Verdünnungsmittels und gegebenenfalls in Gegenwart eines Reaktionshilfsmittels umsetzt; oder daß man

(c) Pyrazolylpyrrolinone der Formel (Ic),

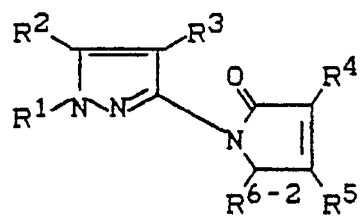

(Ic)

in welcher
$R^{6-2}$ für einen Rest $R^7\text{-}\underset{\underset{O}{\|}}{C}\text{-O-}$
oder für einen Rest $R^8$-$SO_2$-O- steht und
$R^1$, $R^2$, $R^3$, $R^4$, $R^5$, $R^7$ und $R^8$ die oben angegebene Bedeutung haben,
erhält, wenn man die mit Hilfe des erfindungsgemäßen Verfahrens (a) erhältlichen Pyrazolylpyrrolinone der Formel (Ia),

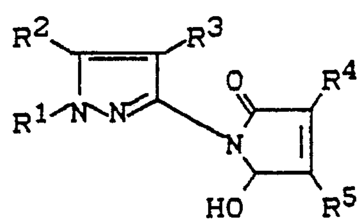

(Ia)

in welcher
$R^1$, $R^2$, $R^3$, $R^4$ und $R^5$ die oben angegebene Bedeutung haben,
entweder
($\alpha$) mit Acylierungsmitteln der Formel (IV),

$R^7\text{-}\underset{\underset{O}{\|}}{C}\text{-}E^1$ (IV)

in welcher
$E^1$ für eine elektronenanziehende Abgangsgruppe steht und
$R^7$ die oben angegebene Bedeutung hat oder
($\beta$) mit Isocyanaten der Formel (V),

$R^{7-1}\text{-}N=C=O$ (V)

in welcher
$R^{7-1}$ für Alkyl steht oder
($\gamma$) mit Sulfonylierungsmitteln der Formel (VI),

$R^8\text{-}SO_2\text{-}E^2$ (VI)

in welcher
$E^2$ für eine elektronenanziehende Abgangsgruppe steht und
$R^8$ die oben angegebene Bedeutung hat,
jeweils gegebenenfalls in Gegenwart eines Verdünnungsmittels und gegebenenfalls in Gegenwart eines Reaktionshilfsmittels umsetzt.

4. Herbizide Mittel, gekennzeichnet durch einen Gehalt an mindestens einem Pyrazolylpyrrolinon der Formel (I) gemäß den Ansprüchen 1 bis 3.

5. Verfahren zur Bekämpfung von Unkräutern, dadurch gekennzeichnet, daß man Pyrazolylpyrrolinone der Formel (I) gemäß den Ansprüchen 1 bis 3 auf die Unkräuter und/oder ihren Lebensraum einwirken läßt.

6. Verwendung von Pyrazolylpyrrolinonen der Formel (I) gemäß den Ansprüchen 1 bis 3 zur Bekämpfung von Unkräutern.

7. Verfahren zur Herstellung von herbiziden Mitteln, dadurch gekennzeichnet, daß man Pyrazolylpyrrolinonen der Formel (I) gemäß den Ansprüchen 1 bis 3 mit Streckmitteln und/oder oberflächenaktiven Substanzen vermischt.

8. Verbindungen der Formel (IIa)

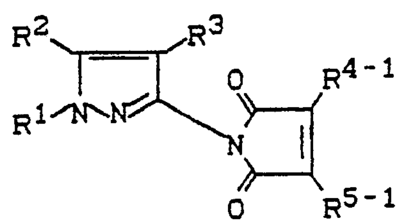

(IIa)

in welcher
$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,
$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,
$R^3$ für Wasserstoff oder Alkyl steht und
$R^{4-1}$ und $R^{5-1}$ entweder unabhängig voneinander jeweils für Wasserstoff oder Alkyl stehen oder gemeinsam für einen zweifach verknüpften Alkandiylrest stehen.

9. Verfahren zur Herstellung von Verbindungen der Formel (IIa)

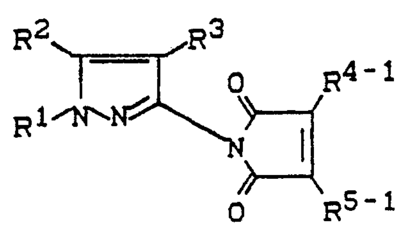

(IIa)

in welcher
$R^1$ für Wasserstoff, Alkyl, Alkenyl, Alkinyl, Halogenalkyl, Halogenalkenyl, Alkoxyalkyl oder für jeweils gegebenenfalls substituiertes Aralkyl oder Aryl steht,
$R^2$ für Wasserstoff, Alkyl, Alkenyl, Alkinyl, Halogenalkyl, Halogenalkenyl, Alkoxyalkyl, Alkylthioalkyl, für gegebenenfalls substituiertes Cycloalkyl oder für gegebenenfalls substituiertes Aryl steht,
$R^3$ für Wasserstoff oder Alkyl steht und
$R^{4-1}$ und $R^{5-1}$ entweder unabhängig voneinander jeweils für Wasserstoff oder Alkyl stehen oder gemeinsam für einen zweifach verknüpften Alkandiylrest stehen,
dadurch gekennzeichnet, daß man Aminopyrazole der Formel (VII)

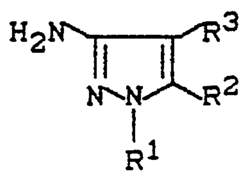

(VII)

in welcher
$R^1$, $R^2$ und $R^3$ die oben angegebene Bedeutung haben,
mit 1,2-Dicarbonsäureanhydriden der Formel (VIII),

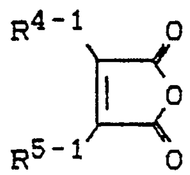

(VIII)

in welcher
$R^{4-1}$ und $R^{5-1}$ die oben angegebene Bedeutung haben,
gegebenenfalls in Gegenwart eines Verdünnungsmittels und gegebenenfalls in Gegenwart eines Acylierungskatalysators umsetzt und gegebenenfalls anschließend so erhältliche Verbindungen der Formel (IIb),

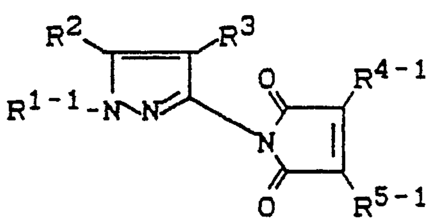

(IIb)

in welcher
$R^2$, $R^3$, $R^{4-1}$ und $R^{5-1}$ die oben angegebene Bedeutung haben und
$R^{1-1}$ für Wasserstoff steht,
am N'-Stickstoffatom des Pyrazolringes mit Alkylierungsmitteln, Alkenylierungsmitteln, Arylierungsmitteln oder Aralkylierungsmitteln gegebenenfalls in Gegenwart eines Verdünnungsmittels und gegebenenfalls in Gegenwart eines Reaktionshilfsmittels umsetzt.

Europäisches Patentamt

# EUROPÄISCHER RECHERCHENBERICHT

Nummer der Anmeldung: EP 89104336.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | DE - A1 - 2 735 841 (CIBA-GEIGY) * Ansprüche 1,12,13; Beispiel 17* -- | 1,4-6 | C 07 D 403/04<br>A 01 N 43/56 |
| A | US - A - 4 169 838 (LANG) * Anspruch 1; Beispiel 20 * -- | 1-9 | |
| A | US - A - 4 643 758 (BUROW) * Ansprüche 1,17 * -- | 1-5 | |
| A | EP - A2 - 0 215 425 (HOECHST) * Ansprüche 1-6 * -- | 1,4,5 | |
| X | CHEMICAL ABSTRACTS, Band 84, Nr. 17, 26. April 1976, Columbus, Ohio, USA KATRITZKY, ALAN R. et al. "Phthaloylation of aminoazoles and aminoazines" Seite 533, Spalte 1, Zusammenfassung-Nr. 121 722e & J. Chem. Soc., Perkin Trans. 1 1976, (3), 309-12 -- | 8 | **RECHERCHIERTE SACHGEBIETE (Int. Cl.4)**<br>C 07 D 403/00 |
| X | CHEMICAL ABSTRACTS, Band 92, Nr. 1, 7. Jänner 1980, Columbus, Ohio, USA DORGAN, RODERICK J.J. et al. "N-Al--kyl and N-acyl derivatives of 3(5)-aminopyrazole" Seite 608, Spalte 2, Zusammenfassung-Nr. 6 458b & J. Chem. Res. Synop. 1979, (6), 198 ---- | 8 | |

Der vorliegende Recherchenbericht wurde für alle Patentansprüche erstellt.

| Recherchenort | Abschlußdatum der Recherche | Prufer |
|---|---|---|
| WIEN | 02-05-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument